# EUROPEAN PATENT APPLICATION

(11) **EP 4 537 741 A1**
(43) Date of publication of application: **16.04.2025**
(21) Application number: 23203424.9
(22) Date of filing: 13.10.2023
(51) Int. Cl.: A61B 5/00, A61B 5/0215, A61B 5/0245, A61B 5/07, A61B 5/29

(54) **INTRAVASCULAR IMPLANT**

(71) Applicant: BIOTRONIK SE & Co. KG, 12359 Berlin (DE)
(72) Inventor: Dörr, Thomas, 12437 Berlin (DE); Wildau, Hans-Jürgen, 10245 Berlin (DE)
(74) Representative: Biotronik Corporate Services SE

(57) **Abstract**

The invention refers to an intravascular implant (100) comprising a blood pressure sensor and an ECG sensor, wherein the blood pressure sensor and the ECG sensor are configured to simultaneously determine blood pressure data and ECG data of the patient, respectively.

## Description

The invention relates to an intravascular implant.

An implant or implantable medical device is an active or passive implantable medical device, for example, a pacemaker (with leads), an Implantable Cardiac Monitor (ICM), an Implantable Leadless Pacer (ILP), an Implantable Leadless Pressure Sensor (ILPS), an Implantable Cardiac Defibrillator (ICD) or a Subcutaneously Implanted Cardiac Defibrillator (S-ICD), a device that delivers spinal cord stimulation (SCS), deep brain stimulation (DBS) or neurostimulation. Such medical device may deliver one or more therapeutic substances, e.g. a drug pump, collect physiological signals in order to monitor the health status of the patient and/or deliver a therapy to the patient, for example using electromagnetic waves. They may be implanted within different organs and body cavities such as the vasculature of the patient.

Implantable pressure sensors which measure blood pressure in patient's body are known, for example, the CardioMEMS^{®} HF system (Abbott) and similar implants. The known system may be implanted in the pulmonary artery to measure the blood pressure. It was shown that the pulmonary artery blood pressure to be a reliable indicator of worsening heart failure, so by monitoring the pulmonary artery blood pressure, the health care practitioner (HCP) can respond rapidly to subtle changes in the heart failure status. By knowing the blood pressure, impending acute decompensation, i.e., the transition from chronic stable heart failure to acute unstable heart failure, can be recognized and thereby prevented by, for example, adjusting the patient's medication.

However, as there are several types of heart failure and different causes, treatment will be improved and disease management will be eased if the HCP would know not only blood pressure measurement data but several heart parameters measured under similar conditions.

Accordingly, there is the need to provide implants which are capable to measure more than one significant and informative parameter.

The above object is solved by the intravascular implant having the features of claim 1.

In particular, the above object is solved by an intravascular implant comprising a blood pressure sensor and an ECG sensor, wherein the blood pressure sensor and the ECG sensor are configured to simultaneously determine actual blood pressure data and actual ECG data of the patient, respectively. Accordingly, the intravascular implant provides a combination of a blood pressure sensor and a sensor that determines an electrocardiogram (ECG). The blood pressure data determined by the pressure sensor and the ECG data are important parameters from which the HCP can evaluate the health status of the patient, in particular with regard to health failure.

The blood pressure sensor is a unit that determines the blood pressure within the blood vessel or other vessel of the patient in which the intravascular implant is located after implantation. The ECG sensor determines ECG data of the patient, i.e. small electrical changes that are a consequence of cardiac muscle depolarization followed by repolarizsatioin during each cardiac cycle (i.e. a period containing one heartbeat), e.g. a P wave representing depolarization of the atria, a QRS complex representing depolarization of the ventricles, and a T wave, which represents repolarization of the ventricles. It is a crucial part of the present invention that the ECG data and the blood pressure data of the blood surrounding the implant at a pre-determined measuring location within the vasculature are determined simultaneously, i.e. at the same time, within a specified time period. For example, the blood pressure data and the ECG data are determined continuously, every minute, every 10 minutes, four times a day or every day. Accordingly, the ECG data and the blood pressure data may be associated with regard to their measuring time point. Hence, the determined ECG data and the blood pressure data may be saved in a data memory of the implant together with an information with regard to the respective measuring time. This means, that the HCP can be certain with regard to the measuring time point of the ECG data and the blood pressure data and may associate these data without any complicated conversion procedure since the ECG data and the blood pressure data use the same data format and origin (the clock unit of the processor of the implant). This is important for the data assessment by the HCP. The HCP can assess them as a single entity.

In an embodiment where the ECG data and the blood pressure data are determined continuously only data may be saved where an irregular heart rhythm is determined.

The simultaneously determination of the ECG data and the blood pressure data may be coordinated by an internal clock, in particular a clock unit of the processor of the implant providing a signal to trigger ECG and blood pressure determination.

The intravascular implant may comprise a hermetically sealed housing and at least one fixation element extending from the housing for fixing / anchoring the implant within the vasculature at the pre-determined measuring location, for example within the pulmonary artery. The housing may comprise an electrical circuitry, a power supply, e.g. a battery or a rechargeable battery, a processor, a data memory, and, in one embodiment, a communication unit. All aforementioned elements are electrically connected and located within the housing. The processor may comprise a clock unit for time-dependent assessment of the data determined by the pressure sensor and the ECG sensor.

In one embodiment, the implant has an elongated housing comprising a first end and a second end, wherein a first fixation element is located, fixed to and extends from the first end and a second fixation element is located, fixed to and extends from at the second end. Such configuration is advantageous because the elongated housing may easily be advanced through the vasculature of the patient using, for example, a catheter and may be arranged at the pre-determined measuring location (target location) of the implant. Dimensions of the housing may be, for example, between 5 mm and 40 mm (length), between 0,5 mm and 3 mm (height) and between 0,5 mm and 3 mm (thickness), wherein the length is the greatest dimension of the housing. Height and thickness are dimensions extending perpendicular to the length. Additionally, the first fixation element and/or the second fixation element may be adapted such that it is flexible and/or takes an elongated configuration for implantation and movement through the vasculature of the patient and a deployed configuration for fixation at the pre-determined location for measuring blood pressure and ECG data within the vasculature. For example, the first fixation element and/or the second fixation element may comprise or consist of at least one material of the group comprising Nitinol and Titanium alloy.

In one embodiment, the first fixation element and/or the second fixation element has a loop-like structure. In particular, the loop-like structure may be located at the first end or second end of the housing. The loop may be a closed loop, wherein the closed end of the respective loop may be located at and fixed to the first end and second end of the housing, respectively. The circular end or bight of the loop may be located at the end of the loop-like structure furthest away from the housing of the intravascular implant. As indicated above, the first fixation element and/or the second fixation element may take an elongated configuration and a deployed configuration, wherein in the elongated configuration the respective loop may be elongated and in the deployed configuration the respective loop may provide a form that is similar to a circular or elliptical form and may adapt to the form of and pressed to the inner vessel wall at the pre-determined location for sensing within the vasculature of the patient. For example, the diameter of the loop in the deployed configuration may be, for example, between 0.1 mm and 3 mm, e.g., between 0.3 mm and 2 mm. In an embodiment, both fixation elements comprise such loop-like structure. Alternatively, the first fixation element and/or the second fixation element may comprise a stent-like structure or wire having an expanded and a contracted configuration for retaining the vascular implant within the vessel. With regard to all embodiments of the fixation element it is noted that the blood flow through the vessel is only slightly hindered at the pre-determined measuring location because the lumen of the blood vessel remains essentially free from the first fixation element and the second fixation element. The material of the first fixation element and/or the second fixation element may comprise or consist of at least one material of the group comprising flexible biocompatible material and a shape memory effect material, for example, Nitinol.

In one embodiment, a section of the loop-like structure of the first fixation element and/or the second fixation element comprises an electrode of the ECG sensor or function as electrode of the ECG sensor. For example, the first fixation element and/or the second fixation element may comprise an electrically conducting and exposed section, for example, if it is formed as a loop, at its bight. The dimension of the electrode may be, for example, between 1mm and 30mm, in particular between 5 mm and 25 mm, in particular between 10 mm and 20 mm. The material of the electrode may contain or consist of at least one material of the group comprising Titanium alloy, e.g. Nitinol. The electrode is electrically connected to the circuitry within the housing of the implant via a feedthrough guiding the connection through the housing wall thereby providing the electrical signals detected by the respective electrode to the processor located within the housing. The feedthrough electrically isolates the respective fixation element from the housing. In a deployed configuration and arrangement of the implant at the pre-determined measuring location within the vasculature of the patient, ECG data may be derived from one electrode located at one of the fixation loops of the first and second fixation element and/or from the electrically conducting housing that may act as a counter electrode. The processor located within the housing and the circuitry form further elements of the ECG sensor.

Accordingly, in one embodiment, the housing comprises an electrode of the ECG sensor at its surface (i.e. the housing acts like an electrode or forms this electrode), wherein the electrode is exposed to the surrounding blood if it is arranged within the vasculature at the pre-determined measuring location. The electrode is, for example, formed by an electrically conducting area of the housing having a two-dimensional dimension between 1mm² to 25mm². The electrode is electrically connected to the circuitry located within the housing. The housing may comprise an electrode arranged thereon, or the housing may be made of an electrically conducting material coated with an electrically insulating material whereby the electrode is formed by a part of the housing not coated with the insulating material. Parylene may be used as insulating material.

To summarize, the ECG data may be derived from one of the following combinations:
- One of the two fixation elements functioning as electrode and the respective other electrode functioning as counter electrode
- One of the two fixation elements functioning as electrode and the housing or at least a part of the housing functioning as counter electrode
- One of the two fixation elements comprising an electrode and the respective other electrode comprising the counter electrode
- One of the two fixation elements comprising an electrode and the housing or at least a part of the housing functioning as counter electrode
- One part of the housing functioning as electrode and another electrically isolated part of the housing functioning as counter electrode

In one embodiment, the blood pressure sensor comprises a membrane forming one section of an outer surface of the housing. For example, the housing and the membrane may consist of Titanium or Titanium alloy and form a hermetically sealed Titanium or Titanium alloy capsule. The membrane may be welded to the housing covering a respective through hole within the housing. The membrane may have a thickness of 10 µm to 50 µm. The membrane conducts the pressure signal provided by the surrounding blood, for example, to a pressure transducing medium, e.g. an oil filling, located within the housing. The membrane directly contacts the pressure transducing medium. In the embodiment, the housing may be filled with silicone oil acting as pressure transducer. By the pressure transducing medium the pressure signal is transduced to a pressure sensor chip (e.g. a piezo-sensor, a capacitive sensor) located within the housing of the implant, wherein the pressure transducing medium is in direct contact with the pressure sensor chip, as well. It may transmit a pressure range of 500 hPa to 1,300 hPa. In one embodiment, the housing comprises two expansion grooves for compensation of pressures changes caused by thermal effects. Accordingly, the blood pressure sensor may be formed by the membrane, the pressure transducing medium, the pressure sensor chip and the processor as well as the circuitry form the blood pressure sensor.

In one embodiment, the first fixation element and/or the second fixation element comprises or consists of a biocompatible material providing a shape memory effect, for example Nitinol. Further, the first fixation element and/or the second fixation element and/or the housing may partially comprise a biocompatible coating, for example, Parylene.

In one embodiment, the data memory may store the determined ECG data and/or blood pressure data and may include any volatile, non-volatile, magnetic, or electrical media, such as a random-access memory (RAM), read-only memory (ROM), non-volatile RAM (NVRAM), electrically-erasable programmable ROM (EEPROM), flash memory, or any other memory device.

In one embodiment, the intravascular implant comprises a communication unit. The communication unit may be adapted for data communication with an external relay device and/or an external computing unit (server). For example, communication may be provided wirelessly ("over the air") using electromagnetic waves, for example Bluetooth, WLAN, ZigBee, NFC, Wibree or WiMAX or MICS (Medical Implant Communication System) in the radio frequency region, or IrDA or free-space optical communication (FSO) in the infrared or optical frequency region. The communication may be one-directional (from the implant to the external relay device or computing unit) or bi-directional. Accordingly, the communication unit may comprise a respective transmitter or a transceiver, e.g. an antenna, to send the determined ECG data and/or blood pressure data to the relay device and/or computing unit. The first fixation element and/or the second fixation element may be adapted to function as an antenna and/or the housing may be adapted to function as an antenna. Accordingly, the respective fixation element and/or the housing may be coupled to the communication unit. In one embodiment, the communication unit may be adapted to receive at least one parameter value for the patient-specific operation of the implant. The implant may execute its program based on the transmitted at least one parameter value.

In one embodiment, the implant may be connected with a remote database via the communication unit, wherein the remote database provides manual or automatic monitoring of blood pressure data and ECG data values provided by the implant. If the patient faces any critical situation, the remote database may contact an HCP based on the received ECG data values and/or blood pressure data values.

In one embodiment, the implant may comprise a motion sensor configured to determine the posture of the patient. The implant may be further configured to determine ECG data and blood pressure data depending on the patient posture at least once a day.

In one embodiment, the implant is introduced in a minimal-invasive fashion through a peripheral venous access through the vasculature to the pre-defined measuring location using an implantation kit. The implantation kit includes an introducer and a steerable implantation catheter. The introducer is a standard component with which access in the peripheral vein, e.g., the femoral vein is created. The implantation catheter is inserted over a guidewire through the introducer and advanced to target location. The implantation catheter surrounds the implant. During implantation the first fixation element and/or the second fixation element take their elongated or compressed configuration to minimize the insertion diameter.

The present invention will now be described in further detail with reference to the accompanying schematic drawing, wherein

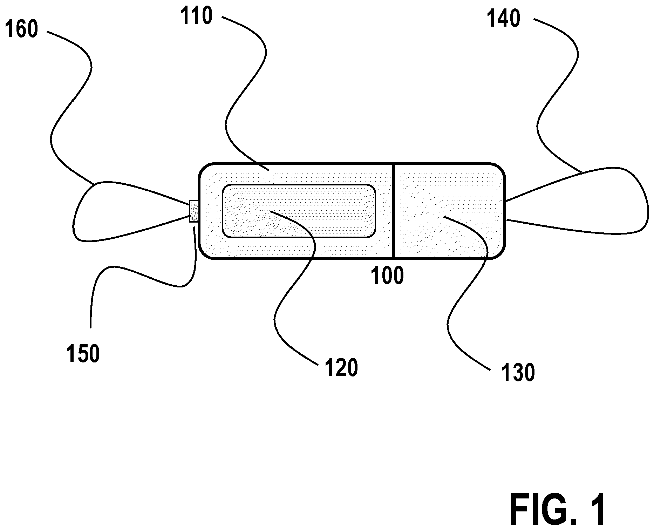

Fig. 1 shows an embodiment of the inventive intravascular implant in a side view.

The embodiment of an intravascular implant 100 depicted in Fig. 1 comprises a hermetically sealed housing 110. One section of the housing 110 is formed by a membrane 120 for transmitting the blood pressure into the housing 110 when implanted. The housing 110 and the membrane 120 consist of a Titanium alloy. Within the housing 110 the implant 100 comprises an electrical circuitry, a battery that is located within a part 130 shown on the right hand side of the housing 110, a processor, a data memory and a communication unit. The electrical circuitry, the battery, the processor, the data memory, and the communication unit are electrically connected to each other. Further, the implant 100 comprises a first fixation loop 140 and a second fixation loop 160, wherein the first fixation loop 140 is mechanically connected to a first end of the elongated housing 110 and the second fixation loop 160 to a second end of the housing 110, located opposite the first end. The first fixation loop 140 and the second fixation loop 160 provide a mechanical fixation of the implant at a pre-defined measuring location within the patient's vasculature. The first fixation loop 140 and the second fixation loop 160 consist of Nitinol.

The housing 110 is further filled with silicone oil that directly contacts the membrane 120 thereby transducing the pressure signal to a piezo sensor located within the housing 110. The silicone oil is in direct contact with the piezo sensor. The piezo sensor transmits the received pressure signals to the processor. Further, ECG data are determined by the second fixation loop 160 forming a first electrode and the housing 110 forming the counter electrode. The electrical signals are transmitted to the processor within the housing 110, as well, wherein the second fixation loop 160 is electrically isolated from the housing by feedthrough 150. The electrical signals of the second fixation loop 160 are transmitted to the processor through the feedthrough 150. The processor comprises a clock unit so that the time point of the measured ECG data and the measured blood pressure data may be stored within the data memory.

After implantation of the implant 100 to the pre-determined measuring location within the patient's vasculature the blood pressure data and ECG data may be determined simultaneaously, i.e. one blood pressure value and one ECG value at the same time. Then, the communication unit may transmit the measured data (including the associated measurement time point) to an external server using, for example, Bluetooth. There, the measured data may be assessed by an HCP. In particular, the HCP may assess the blood pressure data as a unit as they were measured simultaneously. With regard to data communication, the first fixation loop 140 may be used as an antenna.

Accordingly, the inventive implant 100 makes it possible to measure two significant health-related parameters, namely blood pressure and ECG, at the same time and at the same location. This allows a more sophisticated assessment of these data, in particular with regard to heart failure or hypertension (in particular if the implant is implanted within the portal vein. Other potential applications of the invention may be the treatment of vascular heart disease, ischemic heart disease, chronic obstructive pulmonary disease, obstructive sleep apnea or chronic kidney disease.

## Claims

**1.** Intravascular implant (100) comprising a blood pressure sensor (120) and an ECG sensor (110, 160), wherein the blood pressure sensor and the ECG sensor are configured to simultaneously determine blood pressure data and ECG data of the patient, respectively.

**2.** Intravascular implant (100) of claim 1, wherein the implant (100) has an elongated housing (110) comprising a first end and a second end, wherein a first fixation element (140) is located at the first end and a second fixation element (160) is located at the second end.

**3.** Intravascular implant (100) of any one of the previous claims, wherein the first fixation element (140) and/or the second fixation element (160) is adapted such that it takes an elongated configuration and a deployed configuration.

**3.** Intravascular implant (100) of any one of the previous claims, wherein the first fixation element (140) and/or the second fixation element (160) has a loop-like structure.

**4.** Intravascular implant (100) of claim 3, wherein a section of the loop-like structure of the first fixation element and/or the second fixation element comprises an electrode of the ECG sensor.

**5.** Intravascular implant (100) of any one of the previous claims, wherein the housing (110) comprises an electrode of the ECG sensor at its surface.

**6.** Intravascular implant (100) of any one of the previous claims, wherein the blood pressure sensor comprises a membrane (120) forming one section of an outer surface of the housing (110).

**7.** Intravascular implant (100) of any one of the previous claims, wherein the first fixation element (140) and/or the second fixation element (160) comprises or consists of a biocompatible material and/or a material having a shape memory effect, for example Nitinol.

**8.** Intravascular implant (100) of any one of the previous claims, further comprising a power supply, e.g. a battery or a rechargeable battery.

**9.** Intravascular implant (100) of any one of the previous claims, further comprising a communication unit.
